# FASCICULE DE BREVET EUROPEEN

(11) **EP 1 104 489 B1**
(45) Date de publication et mention de la délivrance du brevet: **31.10.2007**
(21) Numéro de dépôt: 99936724.6
(22) Date de dépôt: 11.08.1999
(51) Int. Cl.: C12Q 1/68, C12N 15/00, C12N 15/63

(54) **PROCEDE DE SEPARATION ET DE CARACTERISATION DES FONCTIONS POTENTIELLEMENT PRESENTES DANS UN ECHANTILLON BIOLOGIQUE CONTENANT DES ACIDES NUCLEIQUES**
VERFAHREN ZUR ISOLIERUNG UND CHARAKTERISIERUNG POTENTIELLER FUNKTIONEN AUSGEHEND VON EINER BIOLOGISCHEN PROBE, WELCHE NUKLEINSÄUREN ENTHÄLT
METHOD FOR SEPARATING AND CHARACTERISING FUNCTIONS POTENTIALLY PRESENT IN A BIOLOGICAL SAMPLE CONTAINING NUCLEIC ACIDS

(30) Priorité: 12.08.1998 FR 9810337
(43) Date de publication de la demande: 06.06.2001
(73) Titulaire: Proteus S.A., 30000 Nîmes (FR)
(72) Inventeur: DUPRET, Daniel, F-30420 Calvisson (FR); MASSON, Jean-Michel, F-31400 Toulouse (FR); LEFEVRE, Fabrice, 30900 Nîmes (FR)
(74) Mandataire: Breese, Pierre
(86) Numéro de dépôt international: PCT/FR1999/001972
(87) Numéro de publication internationale: WO 2000/009747

(56) Documents cités:
- WO-A-86/05803
- WO-A-94/24303
- WO-A-98/49275
- US-A- 5 571 690
- SAMBROOK J ET AL.: "Molecular Cloning: A laboratory Manual" 1989 , COLD SPRING HARBOUR LABORATORY PRESS , COLD SPRING HARBOUR XP002102862 * spécialement les chapitres 1,7-9,12,16-18 * le document en entier
- STRATAGENE: "STRATAGENE: 1995 CATALOG" 1995 , STRATAGENE USA XP002102863 page 284 -page 285
- CRAIG D ET AL: "Plasmid cDNA-directed protein synthesis in a coupled eukaryotic in vitro transcription-translation system" NUCLEIC ACIDS RESEARCH, vol. 20, no. 19, 1 janvier 1992 (1992-01-01), pages 4987-4995, XP002088195
- BOWERS T J ET AL: "Isolation, sequencing and expression of the gene encoding a major protein from the bacteriophage associated with Bartonella henselae" GENE: AN INTERNATIONAL JOURNAL ON GENES AND GENOMES, vol. 206, no. 1, 5 janvier 1998 (1998-01-05), page 49-52 XP004102871
- PROMEGA CORPORATION: "PROMEGA: 1997 CATALOG" 1997 , PROMEGA CORPORATION , WOODS HOLLOW ROAD XP002102864 page 2 page 117 -page 136
- MACKOW E R ET AL: "DNA AMPLIFICATION-RESTRICTED TRANSCRIPTION-TRANSLATION: RAPID ANALYSIS OF RHESUS ROTAVIRUS NEUTRALIZATION SITES" PROCEEDINGS OF THE NATIONAL ACADEMY OF SCIENCES OF USA, vol. 87, no. 2, 1 janvier 1990 (1990-01-01), pages 518-522, XP000095665
- PRATT J M: "Transcription and Translation. A practical Approach. Chapter 7: Coupled transcription-translation in procaryotic cell-free systems" 1992 , IRL PRESS , OXFORD XP002102865 cité dans la demande page 179, alinéa 1 -page 209, alinéa 3
- SOUTHWORTH M W ET AL.: "Control of protein splicing by intein fragment reassembly" THE EMBO JOURNAL, vol. 17, no. 4, 1998, pages 918-926, XP002121550
- SOUTHWORTH M W ET AL: "CLONING OF THERMOSTABLE DNA POLYMERASES FROM HYPERTHERMOPHILIC MARINE ARCHEA WITH EMPHASIS ON THERMOCOCCUS SP. 9 N-7 AND MUTATIONS AFFECTING 3'-5' EXONUCLEASE ACTIVITY" PROCEEDINGS OF THE NATIONAL ACADEMY OF SCIENCES OF USA, vol. 93, no. 11, 28 mai 1996 (1996-05-28), pages 5281-5285, XP000652319 ISSN: 0027-8424
- NIEHAUS F ET AL: "Cloning and characterisation of a thermostable alpha-DNA polymerase from the hyperthermophilic archaeon Thermococcus sp. TY" GENE: AN INTERNATIONAL JOURNAL ON GENES AND GENOMES, vol. 204, no. 1-2, 19 décembre 1997 (1997-12-19), page 153-158 XP004100707 ISSN: 0378-1119
- OHUCHI S ET AL.: "In vitro method for the generation of protein libraries using PCR amplification of a single molecule and coupled transcription/translation" NUCLEIC ACIDS RESEARCH, vol. 26, no. 19, 1998, pages 4339-4346, XP002121551
- Resto et al., Nucleic Acids Research, Vol. no. 20, No. 22, 1992, pp. 5979-5983
- THEO LANGE: "Cloning gibberellin dioxygenase genes from pumpkin endosperm by heterologous expression of enzyme activities in Escherichia coli" PNAS, vol. 94, 1997, pages 6553-6558,
- JERMUTUS ET AL: "Recent advances in producing and selecting functional proteins by using cell-free translation" CURRENT OPINION IN BIOTECHNOLOGY, vol. 9, pages 534-548,

## Description

La présente invention a pour objet un procédé selon la revendication 1, permettant de séparer les fonctions potentiellement présentes dans un échantillon biologique contenant des acides nucléiques et de caractériser lesdites fonctions, par expression protéique *in vitro* après transcription et traduction de fragments d'ADN. La caractérisation d'une fonction selon le procédé de l'invention peut se faire entre autre par l'analyse biochimique de cette fonction et éventuellement par le clonage ou le séquençage de la séquence polynucléotidique correspondant à chacune des fonctions éventuellement présentes dans l'échantillon étudié.

Ainsi, le procédé selon la présente invention permet tout particulièrement l'identification et l'isolement de gènes ou des protéines pour lesquelles ils codent possédant une fonction déterminée ou déterminable.

La recherche de nouveaux gènes et/ou des protéines pour lesquelles ils codent constituent un objectif majeur de nombreux laboratoires de biologie moléculaire. En effet, la thérapie génique et cellulaire ou la création d'animaux et de plantes transgéniques, suscitent de nouveaux espoirs pour la santé, le diagnostic et l'alimentation humaine ou animale, mais nécessitent entre autres, d'identifier de nombreux gènes et/ou des activités protéiques. Ainsi, il existe de nombreuses techniques d'isolement et de criblage de gènes par clonage et expression.

Une de ces technique appelée génomique consiste à séquencer une partie ou la totalité du génome d'un organisme, puis à rechercher par homologie avec des séquences déjà identifiées dans des banques de données des séquences potentiellement codantes. Une fois identifiées, ces séquences doivent être sous clonées et exprimées pour vérifier qu'elles codent effectivement pour la propriété recherchée. Outre le temps nécessaire à sa mise en oeuvre, cette technique présente l'inconvénient de ne pouvoir identifier que des fonctions homologues à celles qui sont déjà connues et référencées dans les bases de données.

La protéomique est une deuxième approche possible pour rechercher des propriétés intéressantes. Elle consiste à extraire les protéines exprimées par un microorganisme et à les purifier. Chaque fraction de purification est alors testée pour détecter laquelle contient la propriété recherchée. L'inconvénient majeur de cette technique réside dans le fait qu'elle ne permet pas d'avoir une liaison directe entre la propriété détectée et le gène qui permet l'expression de cette propriété. Le deuxième inconvénient de cette approche est qu'elle ne permet pas de détecter les propriétés qui n'ont pas été induites dans le microorganisme du départ.

Une troisième technique pour détecter une fonction exprimée par un microorganisme consiste à utiliser le clonage d'expression. Cette méthode a pour principe d'extraire l'ADN du microorganisme de départ, à le fragmenter et à l'insérer dans un vecteur d'expression in vivo qui est transformé dans un hôte. Cet hôte est choisi pour sa capacité à exprimer les gènes du microorganisme de départ. De la même façon le vecteur est toujours choisi pour sa compatibilité avec l'hôte d'expression. La technique de clonage d'expression est utilisable dans le cadre de la recherche d'une fonction dans un microorganisme ayant des caractéristiques génétiques proches d'un des hôtes existant (même profil d'usage des codons, même pourcentage GC, ...). Dans le cas de la recherche d'une fonction à partir d'un grand nombre de microorganismes d'origine génétiques variées, la méthode du clonage d'expression devient inutilisable, les hôtes n'étant plus capables d'exprimer les gènes hétérologues qui leur sont fournis.

De plus, le clonage d'expression comme les autres procédés de l'art antérieur d'isolement et de criblage de gènes présentent de nombreux inconvénients :
- la toxicité cellulaire des produits de transcription et de traduction, qui peuvent induire des recombinaisons génétiques pour pallier à ces problèmes de toxicité,
- la faible représentativité de la banque utilisée,
- le temps de mise en oeuvre
- la faible compatibilité entre la séquence du gène cloné et la ponctuation d'expression du vecteur utilisé,
- des niveaux d'expression très variables,
- des problèmes d'usage des codons,
- des problèmes de repliement, de modifications post-traductionnelles,
- le problème posé par l'influence de l'état physiologique d'une cellule sur le niveau d'expression des protéines lorsqu'on cherche l'activité protéique directement dans des extraits cellulaires d'origine,
- une grande difficulté d'automatisation.

La présente invention vise précisément à pallier ces inconvénients en offrant une méthode rapide et efficace d'identification d'une fonction associée à une séquence polynucléotidique contenue dans un échantillon biologique contenant des acides nucléiques, et permettant d'isoler ladite séquence.

Ce but est atteint grâce à un procédé de séparation et de caractérisation des fonctions potentiellement présentes dans un échantillon biologique contenant des acides nucléiques, caractérisé en ce qu'il comprend les étapes suivantes :
a) la détermination de la fonction à cribler ,
b) la préparation de fragments d'acide nucléique à partir dudit échantillon,
c) l'association de chacun desdits fragments avec une molécule vectrice,
d) l'isolement de chaque fragment associé avec une molécule vectrice ou d'une partie de chaque construction constituée par un fragment associé avec une molécule vectrice, de l'étape (c)
e) le traitement dans un système acellulaire de chaque fragment associé avec une molécule vectrice ou d'une partie de chaque construction constituée par un fragment associé avec une molécule vectrice de l'étape (d) pour obtenir des transcrits,
f) le test de la fonction déterminée à l'étape (a) des transcrits obtenus à l'étape (e) ou des protéines pour lesquels ils codent après traduction desdits transcrits.

Le procédé de l'invention offre l'avantage de pouvoir réaliser :
- un test des propriétés des transcrits de l'étape (e), lorsque ceux-ci présentent des propriétés avantageuses de type tRNA, rybozyme, ou
- un test des propriétés des protéines codées par lesdits transcrits.

Dans le cas où l'on teste les fonctions des protéines codées par les transcrits obtenus à l'étape (e) le procédé de l'invention comprend à l'étape (f) le traitement desdits transcrits *in vitro* avec un extrait cellulaire permettant leur traduction en protéine, puis le test d'une fonction desdites protéines par tout moyen approprié.

On entend plus particulièrement par fonction au sens de la présente invention, la propriété qui peut être codée par une séquence polynucléotidique. Cette propriété peut être par exemple une activité enzymatique ou une affinité si ladite séquence code pour une protéine, ou une activité endonucléase par exemple si ladite séquence code pour un ARNm catalytique.

Ainsi, le procédé de l'invention permet non seulement de détecter une fonction, mais aussi de la caractériser d'un point de vue biochimique. Si la fonction est une activité enzymatique par exemple, il peut s'agir de l'analyse des conditions optimales de fonctionnement (pH, température, concentration en sels), des paramètres cinétiques (Vm, Km), des paramètres d'inhibition (Ki). Si la fonction est une affinité, il peut s'agir de la détermination du Kd, ou de la molécule ayant le plus d'affinité pour cette protéine. Il peut s'agir encore de la détermination de la taille de la protéine traduite ou de l'ARNm transcrit, et éventuellement du séquencage du gène correspondant.

On entend par échantillon biologique tout échantillon susceptible de contenir des acides nucléiques, comme un échantillon de sol, de végétal, de sang, humain ou animal, d'eau, de culture microbienne, cellulaire ou virale, de biopsie, etc..., mais ces échantillons peuvent également correspondre à des produits d'amplification (PCR, NASBA, etc...), de l'ADN génomique, de l'ADN synthétique, de l'ARNm, ou tout produit d'acides nucléiques issus de traitements couramment utilisés par l'homme de métier.

On entend par molécule vectrice, une ou plusieurs séquences polynucléotidiques comportant au moins un promoteur de transcription pour l'étape (e) et éventuellement une substance facilitant l'isolement du fragment à l'étape (d). Avantageusement cette substance peut être une ou plusieurs molécules de streptavidine ou de biotine, de groupement polypyrol, d'anticorps, une séquence polynucléotidique simple ou double brin, un vecteur d'ADN plasmidique ne comportant préférentiellement pas de séquences permettant l'expression *in vivo* du fragment associé, ou toute autre composé permettant l'isolement du fragment associé à l'étape (d).

On entend par isolement à l'étape (d) du procédé de l'invention la subdivision du lot de fragments obtenus a l'étape (c) en sous-ensembles, un sous-ensemble pouvant être constitué d'un seul ou de plusieurs fragments d'acide nucléique.

L'étape (b) de préparation de fragments d'acides nucléiques du procédé de l'invention, peut comprendre une phase d'extraction si les acides nucléiques ne sont pas directement accessibles dans l'échantillon biologique, par exemple dans le cas d'acides nucléiques contenus dans des cellules, des virus, du sang, des éléments organiques, etc... . Cette phase d'extraction est alors incluse dans l'étape (b) de préparation de fragments d'acides nucléiques. De même dans le cas où l'échantillon biologique est constitué d'ARNm, une étape de RT-PCR est nécessaire pour préparer les fragments d'acides nucléiques de l'étape (b).

Selon une forme de mise en oeuvre particulière du procédé de l'invention, la molécule vectrice est composée de deux séquences polynucléotidiques comportant chacune au moins un promoteur de transcription. Chacune de ces séquences est associée à une extrémité d'un des fragments obtenus à l'étape (b).

Selon une forme de mise en oeuvre avantageuse du procédé de l'invention, le(s) promoteur(s) de transcription porté(s) par la molécule vectrice est (sont) un (des) promoteur(s) de type fort(s), tel que le promoteur de transcription de la RNA polymérase du phage T7, SP6, Qβ ou λ.

Ainsi, une application particulière du procédé de l'invention permet d'identifier une séquence polynucléotidique et/ou la protéine correspondante, possédant une fonction, à partir d'un échantillon contenant des acides nucléiques.

On entend par vecteur recombinant dans l'application du procédé de l'invention ci-dessus, un vecteur, par exemple plasmidique, dans lequel a été introduit un fragment, et par partie de ce vecteur, la partie du vecteur recombinant contenant le fragment obtenu à l'étape (b) et les éléments nécessaires à la mise en oeuvre des étapes et (e) et (f).

Les étapes du procédé de l'invention peuvent être réalisées successivement sans interruption par le même opérateur, avantageusement sur un dispositif automatisé intégrant chacune des étapes, ou peuvent être réalisées de façon discontinues, éventuellement par des opérateurs différents.

L'étape (e) de transcription et la phase de traduction de l'étape (f) des fragments sont aussi désignées conjointement réaction d'Expression Protéique In Vitro, désigné aussi réaction EPIV. La réaction EPIV peut être simultanée, ce qui signifie que la phase de traduction de l'étape (f) est réalisée simultanément avec la transcription de l'étape (e), ou décomposée en deux étapes distinctes, (e) de transcription et (f) de traduction.

Le découplage des étapes (e) et (f) permet d'optimiser les rendements de chaque étape, et ainsi de produire des quantités plus importantes de protéines, ce qui trouve toute son utilité dans le cas d'enzymes de faible activité spécifique.

Ce découplage permet aussi de normaliser la formation des produits à l'étape (f) et de pouvoir comparer ultérieurement les différentes fonctions exprimées.

Le découplage entre la transcription de l'étape (e) et la traduction de l'étape (f) permet également d'éviter les problèmes de dégradation de la matrice ADN par les nucléases si celle-ci a été préparée par PCR. En effet, les composants de la réaction de transcription sont moins contaminés par des nucléases, contrairement aux extraits de traduction.

Le découplage permet en outre l'emploi d'extraits de traduction différents selon l'origine de l'ADN criblé. En effet, la phase de traduction du transcrit à l'étape (f) est avantageusement réalisée avec un extrait de traduction de la même origine ou d'une origine proche de celle de l'échantillon biologique sur lequel est pratiqué le procédé de l'invention. Ainsi, on optimise l'adéquation entre l'origine des signaux de traduction des transcrits et l'extrait cellulaire pour une efficacité de traduction optimale. On peut citer à titre d'exemple, l'utilisation d'un extrait de traduction d'un organisme extrêmophile pour le criblage d'une banque d'ADN du même organisme ou d'un autre organisme extrêmophile (thermophiles, halophiles, acidophiles, etc...) ou encore un extrait de traduction de cellules eucaryotes pour le criblage d'une banque d'ADN eucaryote. Ces extraits respectifs sont susceptibles d'améliorer l'efficacité du procédé. Ces extraits sont choisis pour leur capacité à traduire les transcrits à l'étape (f).

Le procédé de l'invention est remarquable en ce qu'il met en oeuvre une adéquation entre la ponctuation d'expression des transcrits de l'étape (e) et les extraits de traduction utilisés. Ces extraits sont aussi caractérisés en ce que soit ils ne contiennent pas la propriété recherchée, soit ils la contiennent mais qu'elle n'est pas détectable dans les conditions de test réalisées pour détecter la fonction recherchée. Il s'agit par exemple de l'utilisation d'un extrait de traduction contenant une activité beta-galactosidase mésophile permettant de traduire un ARNm d'une beta-galactosidase thermophile et de la détection de l'activité de cette dernière à haute température, ce qui élimine l'activité beta-galactosidase mésophile.

En fonction de l'origine génétique des fragments obtenus à l'étape (b), par exemple ADN de microorganismes Gram positifs, ou négatifs, d'eucaryotes, de virus etc..., et de la fonction testée, différents extraits de traduction peuvent donc être utilisés.

Une mise en oeuvre particulière du procédé de l'invention consiste à utiliser à l'étape (f) un extrait de traduction qui soit en fait un mélange de plusieurs extraits de traduction. Il peut s'agir par exemple d'extrait de traduction de *E*. *coli* surexprimant une protéine chaperon A mélangé avec un extrait de traduction de *E*. *coli* surexprimant une protéine chaperon B. Tout type de mélange est envisageable du moment qu'il correspond aux caractéristiques décrites ci-dessus. De la même manière, il est possible d'utiliser un extrait de traduction dans lequel est rajouté un ou plusieurs tRNAs spécifiques d'un ou de plusieurs codons. Les extraits de traduction ainsi obtenus permettent alors de traduire des mRNA comportant ces codons spécifiques, comme par exemple la traduction d'un mRNA contenant un codon ambre en rajoutant dans l'extrait de traduction un ou des tRNAs suppresseurs.

Le traitement de l'étape (f) avec un extrait de traduction peut aussi être réalisé avec un extrait universel de traduction quelque soit l'origine de l'échantillon comme par exemple un extrait d'*E. coli* et/ou tout(s) autre(s) extrait(s) cellulaire(s) supplémentés ou non par des molécules intéressantes comme celles, par exemple, indiquées précédemment (tRNA, chaperon...).

Il est également possible d'ajouter à l'extrait de traduction de l'étape (f) une ou plusieurs substances favorisant un repliement ou une maturation plus efficace des protéines exprimées, comme par exemple des chaperons, des détergents, des sulfobétaïnes, des extraits membranaires, etc....

Le test d'une fonction des protéines synthétisées à l'étape (f) peut être réalisé par tout moyen approprié permettant par exemple de détecter la ou les activités enzymatiques recherchées. Cette forme de mise en oeuvre sera plus particulièrement appliquée lors de la recherche d'enzymes aux propriétés originales, comme par exemple des enzymes thermostables, actives à haute température, en milieu acide, en milieu à forte concentration saline, etc..., à partir d'un échantillon d'ADN issu d'organisme(s) extrêmophile(s). Ces enzymes extrêmophiles, souvent actives dans des conditions proches des conditions physiologiques des souches qui les produisent (températures, salinité, pH, etc...) sont des outils particulièrement intéressants pour de nombreux procédés industriels (industries de l'agro-alimentaire, de la nutrition animale, du papier, des détergents, du textile, etc...), où elles peuvent se substituer à leurs homologues mésophiles.

Le procédé de l'invention offre l'avantage de pouvoir réaliser :
- un test des propriétés des transcrits de l'étape (e), lorsque ceux-ci présentent des propriétés avantageuses de type tRNA, rybozyme, ou
- un test des propriétés des protéines codées par lesdits transcrits.

Dans le cas d'un ribozyme ayant un activité endonucléase par exemple, il est possible de détecter cette activité en utilisant une matrice nucléotidique ayant un groupement fluorescent à une extrémité et un groupement "quencher" de l'autre. En cas de coupure de la matrice par le ribozyme, le groupement fluorescent est séparé du groupement "quencher" ce qui libère la fluorescence du premier.

Dans le cas d'un tRNA, il est possible d'utiliser par exemple une fraction de la réaction contenant potentiellement ce tRNA et de la mettre dans une réaction de traduction *in vitro* contenant le mRNA d'un gène rapporteur dont l'un des codons ne peut être lu que par le tRNA recherché. Si l'activité du rapporteur est détectée, cela signifie que le tRNA était présent dans la fraction initiale et qu'il a permit la traduction in vitro du mRNA du rapporteur

Dans le cadre de la mise en évidence d'une activité enzymatique, tout type de substrat spécifique peut être envisagé par l'homme de métier pour mettre en évidence la présence ou l'absence de la fonction recherchée à l'étape (f) Toute(s) transformation(s) du ou des substrats par la ou les fonctions recherchées peut(vent) être détecté(s) par toute méthode connue de l'homme du métier (fluorimétrie, colorimétrie, absorbance, viscosité, etc...).

Dans le cas de la mise en évidence d'une affinité, il est possible d'utiliser par exemple, en fonction de l'affinité recherchée : anticorps-antigène, protéine fixant l'ADN - ADN, récepteur-ligand, etc..., des tests tels que la fixation de ligands radiomarqués, une détection immunologique comprenant l'immobilisation d'un antigène, sa détection spécifique avec l'anticorps recherché, et la révélation de cet anticorps recherché grâce à un anticorps anti-anticorps couplé à une activité rapporteur pouvant être révélée, ou une détection d'un antigène en utilisant un anticorps de chèvre fixé sur un support capable de reconnaître l'antigène, l'antigène pouvant être détecté par un deuxième anticorps de lapin(formation d'un sandwich) couplé indirectement ou non à une activité rapporteur (type phosphatase alcaline ou péroxydase).

Une forme de mise en oeuvre particulière du procédé consiste à l'étape (f) à tester, en parallèle ou de manière simultanée ou non, différentes propriétés d'une même fonction ou plusieurs fonctions.

Ainsi, le procédé de l'invention est remarquable en ce qu'il permet non seulement la détection des fonctions potentiellement contenues dans un échantillon biologique, mais aussi :
- leur quantification, lorsque ces propriétés le permettent comme par exemple des activités enzymatiques ou des affinités.
- leur caractérisation notamment biochimique, comme par exemple les conditions optimales de fonctionnement en température, pH, concentration saline, etc..., poids moléculaire, séquençage protéique, et éventuellement séquençage de la séquence polynucléotidique correspondante.

Le procédé de l'invention est aussi remarquable en ce qu'il est totalement indépendant de l'expression *in vivo* des protéines. Les hôtes cellulaires, comme des micro-organismes, s'ils sont utilisés, ne le sont que pour l'isolement et l'amplification des fragments hétérologues. En conséquence, le procédé de l'invention permet d'éviter tous les problèmes rapportés précédemment avec les méthodes de clonage par expression de l'art antérieur. En particulier, le procédé de l'invention trouve tout son intérêt dans l'identification d'un gène exprimant une protéine cytotoxique. Dans ce sens, la molécule vectrice associée à chaque fragment de l'étape (c) peut être un vecteur plasmidique ne permettant pas de préférence l'expression dudit fragment *in vivo*. A titre d'exemple d'un tel vecteur on peut citer : pBR322 ou pACYC184.

Avantageusement, les fragments préparés à l'étape (b) du procédé de l'invention sont obtenus par l'action d'une ou plusieurs endonucléases sur les acides nucléiques de l'échantillon biologique ou sur les produits de PCR desdits acides nucléiques. Ces fragments peuvent aussi être obtenus par une action mécanique sur ces acides nucléiques, par exemple par passage dans une aiguille de seringue, disruption sous pression, sonication, etc... .

Dans le mode particulier de réalisation du procédé de l'invention où les acides nucléiques de l'échantillon qui peuvent ne pas nécessiter de fragmentation, comme par exemple des banques génomiques, l'étape (b) consiste à préparer les fragments de cet échantillon pour l'étape (c), par exemple par extraction, et/ou purification et/ou préparation des extrémités pour l'association avec les molécules vectrices, par exemple l'insertion dans le vecteur plasmidique.

Dans une forme particulière de mise en oeuvre du procédé de l'invention, les fragments préparés à l'étape (a) ont une taille de 1 à plusieurs dizaines de kb, préférentiellement de 1 à 40 kb et avantageusement de 1 à 10 kb lorsque l'échantillon provient d'un organisme procaryote. De préférence, les fragments préparés à l'étape (a) ont une taille de l'ordre de 5 kb. En effet, la taille moyenne d'un gène procayote est d'environ 1000 paires de bases. En utilisant des fragments de 5000 paires de bases, il est possible d'obtenir des clones portant le gène complet avec leur propre site de fixation du ribosome. Dans le cas où l'ADN est d'origine eucaryote, la taille des fragments préparés à l'étape (a) est beaucoup plus importante, avantageusement de l'ordre de plusieurs dizaines à plusieurs centaines de kilobases.

On peut noter que les fragments préparés à l'étape (a) peuvent porter un opéron partiel ou entier.

L'échantillon biologique à partir duquel sont préparés les fragments de l'étape (b) peut provenir d'un ou plusieurs organismes procaryotes ou cellules d'eucaryotes, ou encore de virus, identiques ou différents. Il peut s'agir par exemple d'un échantillon d'acides nucléiques d'un micro-organisme ou d'un mélange de micro-organismes, ou de cellules eucaryotes de tissus ou d'organismes identiques ou différents. Mais l'échantillon d'acides nucléiques peut également être constitué d'une séquence ou d'une banque d'acide(s) nucléique(s). L'échantillon biologique peut être constitué d'organismes et/ou d'acides nucléiques connus ou inconnus. L'échantillon peut aussi avantageusement être constitué d'acides nucléiques synthétiques.

Dans le cas d'une banque d'ADN eucaryote, la réaction de transcription peut être complétée par une réaction de *splicing* et de maturation *in vitro* des ARNm en utilisant par exemple un extrait nucléaire (3).

Le procédé de l'invention assure une liaison directe entre la fonction mise en évidence à l'étape (f) et la séquence polynucléotidique correspondante. Ce procédé est donc tout particulièrement destiné à détecter des fonctions et à identifier les gènes correspondant à partir d'ADN génomique. On entend par gène un fragment ou une séquence d'ADN associé à une fonction biologique.

Comme indiqué précédemment, selon une forme de réalisation particulière du procédé de l'invention, la molécule vectrice associée aux fragments d'acide nucléique à l'étape (c) est un vecteur plasmidique. Dans ce cas, à l'étape (c) du procédé de l'invention, chaque fragment est inséré dans un vecteur au niveau d'un site de clonage ou d'une cassette de restriction. Ce vecteur plasmidique est caractérisé en ce qu'il comprend un promoteur d'ARN polymérase d'un côté du site de clonage et éventuellement d'un terminateur d'ARN polymérase de l'autre côté. Il est aussi possible de concevoir un vecteur comprenant un site de clonage encadré par deux promoteurs d'ARN polymérase identiques ou différents et éventuellement flanqués de part et d'autre du ou des terminateurs d'ARN polymérase correspondants. Ces promoteurs et éventuellement terminateurs ont préférentiellement pour caractéristique de ne pas fonctionner dans le micro-organisme pouvant être utilisé pour la séparation des vecteurs recombinants à l'étape (d).

Dans le cas où le vecteur ne possède pas de promoteur(s) et/ou d'éventuel terminateur(s) d'ARN polymérase, ou dans le cas ou les promoteur(s) et éventuels terminateur(s) d'ARN polymérase ne sont pas adéquats pour réaliser l'étape (e), ces promoteur(s) et éventuels terminateur(s) peuvent être insérés à l'étape (d) du procédé de l'invention par tout moyen approprié. Une mise en oeuvre avantageuse de cette insertion consiste à réaliser une PCR avec un jeu d'amorces portant les séquences des promoteur(s) et terminateur(s).

Selon une forme de mise en oeuvre particulière du procédé de l'invention, les promoteur(s) et éventuels terminateur(s) sont de type forts comme par exemple ceux de la T7 ARN polymérase.

Dans le cas, où la molécule vectrice est un vecteur plasmidique, l'isolement du vecteur recombinant à l'étape (d) peut être réalisé par transformation de cellules hôtes par l'ensemble des vecteurs recombinants obtenus à l' étape (c) de façon à créer une banque de clones, puis en ce qu'on réalise une extraction du vecteur ou d'une partie du vecteur recombinant contenu par chaque clone de la banque par tout moyen approprié.

L'extraction du vecteur recombinant ou d'une partie de vecteur recombinant flanquée de promoteur(s) et d'éventuel(s) terminateur(s) d'ARN polymérase peut être réalisée par toute méthode connue de l'homme du métier, comme par mini préparation et éventuellement digestion ou par PCR. Une alternative avantageuse consiste à réaliser cette PCR avec des oligonucléotides protégés en 5' des attaques nucléasiques, notamment des nucléases contenues dans le milieu de traduction, par des groupements phosphorothioates.

Comme indiqué précédemment, l'isolement à l'étape (d) peut être réalisé par tout moyen physique, C mécanique ou chimique comme par exemple une simple dilution extrême de l'ensemble des fragments associés à la molécule vectrice à l'étape (c). Mais l'isolement peut aussi avantageusement être réalisé en utilisant les propriétés d'une substance spécifique incluse dans la molécule vectrice, comme une molécule d'anticorps, et l'isolement du fragment est réalisé en utilisant l'affinité anticorps-antigène, ou une biotine, et l'isolement est réalisé en utilisant l'affinité biotine-streptavidine, etc....

Selon une forme particulière de mise en oeuvre du procédé de l'invention, on effectue un tri des fragments ou partie de ceux-ci associés chacun à une molécule vectrice obtenus à l'étape (d). Pour cela, on réalise une réaction EPIV pour chacun des fragments ou partie de ceux-ci associés à une molécule vectrice obtenus à l'étape (d) en incorporant dans le mélange réactionnel de la phase de traduction de l'étape (f) un marqueur de synthèse protéique (tRNA biotinylé, acide aminé modifié, etc...). Chaque produit de la réaction EPIV est alors analysé, par exemple par ELISA, pour la présence d'une protéine exprimée. Les fragments ou partie de ceux-ci associés à une molécule vectrice pour lesquels la réaction EPIV est négative sont déterminés. Ces fragments ne possèdent pas d'ORF sur leur insert. Suite à cette procédure de pré-criblage, ces fragments sont éliminés des criblages ultérieurs d'identification d'activités liées à une protéine. Une telle procédure de pré-criblage permet un gain de temps et de réactifs dans le cas d'un criblage répétitif, et non simultané de la même banque.

Selon une forme de mise en oeuvre particulière, le procédé de l'invention est réalisé entièrement sur un support solide de type puce ou membrane ou plaque de nanotitration. Le support de type puce peut être une plaque de verre, une membrane de nitrocellulose ou tout autre support connu de l'homme de l'art. Les fragments associés à la molécule vectrice sont isolés sur ce support de type puce ou plaque de nanotitration, et les réactifs permettant de mettre en oeuvre le procédé de l'invention sont déposés sur ce support. Le test de la ou des fonctions recherchées peut être mené directement sur le support après un éventuel lavage de celui-ci. Dans le cas où la molécule vectrice est un vecteur plasmidique et où le procédé de l'invention est réalisé sur un support, les colonies transformées par les vecteurs recombinants sont transférées séparément les unes des autres sur un même support, puis lysées *in situ* (3) afin que chaque colonie puisse libérer sur le support les copies du vecteur recombinant qu'elle contient. Une autre forme de mise en oeuvre du même type consiste à déposer séparément les uns des autres sur un même support chaque vecteur ou partie de vecteur recombinant. Il est alors possible de déposer des réactifs permettant de réaliser une réaction EPIV sur le support comportant l'ADN déposé selon l'une des techniques décrites ci-dessus. Le test d'une fonction peut être mené directement sur le support après un éventuel lavage de celle-ci.

Le procédé de l'invention offre l'avantage par rapport aux méthodes de l'art antérieur d'être automatisable. Dans le cas ou la molécule vectrice est un vecteur plasmidique, cette automatisation peut être menée, par exemple de la façon suivante :
- Chaque vecteur recombinant de la banque constituée à l'étape (c) peut être mis en culture sur un support, dans un puit de micro plaque par un robot de type Colony Picker.
- Cette culture peut servir à une étape d'extraction plasmidique réalisée par un robot de type Biorobot 9600 (QIAGEN), ou à une étape d'amplification PCR mise en oeuvre par une machine de type MultiProbe (PACKARD), sur un thermocycleur automatique de type PTC 200 ou PTC 225 (couvercle robotisé - MJ RESEARCH).
- La purification éventuelle des produits PCR peut être menée par l'automate BioRobot 9600.
- La réaction EPIV des étapes (e) et (f) peut être entièrement gérée par le robot MultiProbe. Les tests des fonctions des transcrits obtenus à l'étape (d) peuvent être effectués sur le robot pipeteur et la lecture des résultats est obtenue sur un lecteur correspondant. Si la réaction de transcription est découplée de la réaction de traduction, la purification éventuelle des ARNm peut être réalisée par le BioRobot 9600.
- Les tests d'activité des protéines synthétisées à l'étape (f) sont effectués par le robot pipeteur, et la lecture des résultats est obtenue sur un lecteur (spectrophotométrie, colorimétrie, fluorimétrie, etc..., en fonction du test réalisé) de micro plaques ou par tout autre moyen approprié.

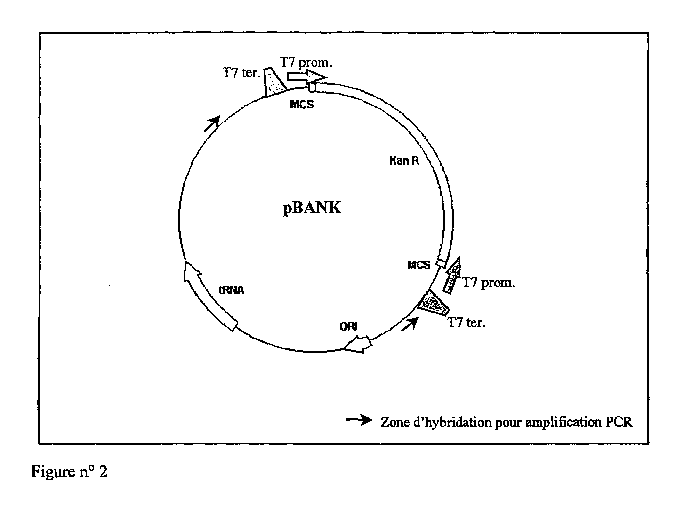

Tout vecteur plasmidique présentant les caractéristiques définies précédemment peut être utilisé dans le procédé de l'invention. On peut citer à titre d'exemple, un vecteur représenté à la figure 2 et construit de la façon suivante :
- Une origine de réplication plasmidique
- Un site de clonage encadré par deux promoteurs identiques, comme celui de la T7 ARN polymérase, ou de tout autre promoteur fort d'ARN polymérase, tel que Qβ, T3, SP6, etc..., et éventuellement flanqués de part et d'autre du terminateur de la même ARN polymérase. Ces promoteurs et terminateur s'il est présent, ne fonctionnent préférentiellement pas dans le microorganisme utilisé pour séparer les vecteurs recombinants. Une telle construction permet de transcrire un fragment d'ADN inséré dans le site de clonage, quel que soit son sens d'insertion. La probabilité de trouver un bon clone est donc multipliée par deux. La taille moyenne d'un gène procaryote est d'environ 1000 pb. En utilisant des fragments d'ADN procaryote de 5000 pb environ pour générer la banque, il est fort probable d'obtenir des clones portant des gènes complets, avec leur propre site de fixation du ribosome (ou RBS pour Ribosome Binding Site). Avec ce système de double promoteur, le gène est situé au pire à 2000 bases du début de l'ARNm, ce qui permet une expression efficace de la protéine correspondante par le procédé de l'invention (comme rapporté dans la partie expérimentale ci-après sur l'activité β-lactamase).
- Eventuellement des séquences spécifiques de part et d'autre des terminateurs, servant de sites d'hybridation pour une amplification par PCR du fragment d'acide nucléique porté par le vecteur.
- Un gène de sélection constitué par un gène de tRNA (4). Éventuellement, en parallèle, un gène de résistance à un antibiotique (ou un autre type de gène de sélection) est inséré dans le site de clonage. Cette sélection antibiotique n'est utilisée que pour l'amplification préparative du vecteur de clonage. En effet, lors de l'insertion de chacun desdits fragments de l'étape (c), un fragment d'ADN se substitue à ce gène de résistance. Ce système présente l'avantage de ne pas dépendre d'une sélection antibiotique, qui pose des problèmes de contamination et de dégradation de l'antibiotique, et permet d'obtenir un vecteur recombinant ne possédant pas d'autre ORF que celle éventuellement introduite par le fragment hétérologue. D'autre part, il permet d'évaluer très rapidement le taux de clones négatifs, en pratiquant un étalement parallèle d'une fraction de la banque sur milieu minimum et sur milieu contenant l'antibiotique de sélection.

Le procédé de l'invention est remarquable en ce qu'il permet de rechercher des fonctions d'acides nucléiques dans des prélèvements bruts de les caractériser et d'identifier la séquence nucléotidique correspondante. En effet, il évite d'isoler chaque microorganisme présent dans ce prélèvement. L'isolement des microorganismes contenus dans un prélèvement est connu pour donner des résultats limités, car seulement quelques pour cent de la diversité des microorganismes présents sont récupérés. En outre, le procédé de l'invention offre un gain de temps considérable. La méthode de l'invention permet de réaliser un meilleur résultat, puisque la biodiversité criblée est de l'ordre de 100%, dans un délai de 5 à 10 jours. Alors que les méthodes de l'art antérieur permettent d'isoler de l'ordre de 5% de la biodiversité présente dans un prélèvement en plusieurs semaines voir plusieurs mois, et autant de temps est nécessaire pour cribler ces souches en vue de détecter une activité protéique testée. Le procédé de l'invention est également avantageux pour rechercher des activités protéiques dans un germe ou une cellule donnée. Une banque génomique ou d'ADNc peut être facilement préparée et criblée selon le procédé de l'invention. Dans le cas d'une banque d'ADNc, la molécule vectrice comprend une séquence d'initiation de la traduction en adéquation avec l'extrait de traduction utilisé à l'étape (f). Outre sa rapidité, le procédé de l'invention permet de s'affranchir de la physiologie cellulaire. Il est ainsi possible de détecter les activités protéiques sans avoir à résoudre des problèmes de culture et d'états physiologiques. Le procédé de l'invention permet de remonter directement au gène à partir de la détection d'une fonction d'un organisme. Il permet aussi de rechercher une fonction dans un organisme contenant potentiellement ladite fonction. Cette technique permet, grâce à sa rapidité et son efficacité, de cribler une ou plusieurs fonctions d'un grand nombre d'organismes d'une collection et donc de valoriser sa biodiversité microbiologique. Ce procédé trouvera toute son utilité dans l'identification d'activités protéiques à intérêt industriel à partir d'organismes extrêmophiles isolés, ou encore d'un prélèvement brut desdits organismes.

Enfin, le procédé de l'invention trouve un intérêt dans le domaine de la génomique. De par sa conception, il permet d'identifier de nouveaux gènes sans passer par le séquençage en premier étape, car il permet de passer directement de la détection d'une fonction à la séquence d'acide nucléique correspondante. Appliqué à la totalité de l'ADN génomique d'un organisme, le procédé de l'invention permet d'accéder à la caractérisation du phénotype total de cet organisme, ce qui introduit la notion de "phénomique".

D'autres avantages et caractéristiques de l'invention apparaîtront des exemples de réalisation de l'invention qui suivent et qui se réfèrent aux dessins en annexe dans lesquels :

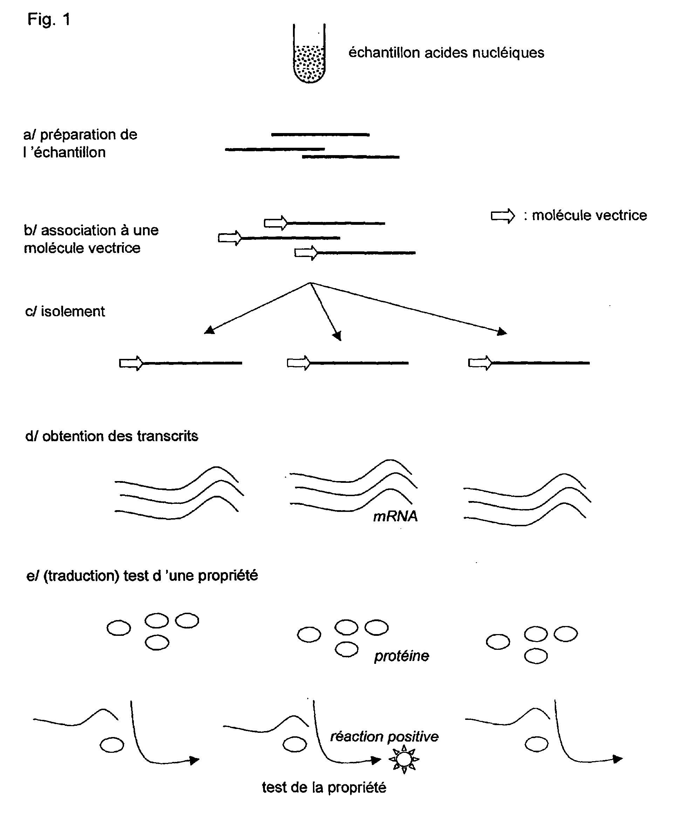

La figure 1 est un représentation schématique d'un exemple de mise en oeuvre du procédé de l'invention.
La figure 2 représente un exemple de carte d'un vecteur de clonage pour la construction d'une banque selon le procédé de l'invention.
La figure 3 représente les cartes des plasmides pADH, pTEM, pET26-Tfu2, pLIPet pGFP utilisés dans le cadre des exemples de réalisation du procédé de l'invention.
La figure 4 représente l'activité de l'intéine 2 d'une ADN polymérase de *Thermococcus fumicolans* (Tfu) produite *in vivo* par clonage d'expression. Piste 1 : échelle 1Kb. Piste 2 : réaction sans enzyme. Piste 3 : réaction avec intéine 2.
La figure 5 représente l'activité de la GFP produite par réaction EPIV (émission de fluorescence suite à une exposition à environ 400 nm) avec des extraits de traduction de mésophile. Tube A : réaction EPIV avec de l'eau (témoin). Tube B : réaction EPIV avec le vecteur pGFP.
La figure 6 représente l'activité de l'intéine 2 de la Tfu ADN polymérase produite par réaction EPIV avec des extrait de traduction de type mésophile. Piste 1 : échelle 1Kb. Piste 2 : T- (réaction sans enzyme). Piste 3 : Blanc (réaction avec extrait EPIV sans ADN). Piste 4 : T+ (réaction avec intéine 2 produite *in vivo*). Piste 5 : Essai (réaction avec intéine 2 produite par EPIV (pET26-Tfu2)).
La figure 7 représente la détection de l'activité d'une enzyme thermophile en utilisant un extrait de traduction mésophile contenant ladite activité. Tube A : réaction EPIV avec de l'eau (témoin) incubée à 37°C, Tube B : réaction EPIV incubée à 37°C, Tube C : réaction EPIV avec de l'eau (témoin) incubée à 70°C après centrifugation, Tube D : réaction EPIV incubée à 70°C après centrifugation.

### I - Matériel.

### 1) Souches et plasmides.

Le vecteur pET26b+ fait partie de la famille des vecteurs pET développée par Studier et Moffatt (8) et commercialisée par la société NOVAGEN. Ce vecteur permet d'exprimer des gènes sous le contrôle du promoteur du phage T7. Le vecteur PINPOINT^{™} (PROMEGA) porte le gène cat (codant pour la chloramphénicol acétyl-transférase) sous contrôle du promoteur de l'ARN polymérase du phage T7. Le vecteur pHS2-22-21 a été construit en introduisant par PCR inverse le site de coupure reconnu par l'intéine 2 de la Tfu ADN polymérase positionné dans le site de polyclonage du plasmide pUC19. Le vecteur pHS2-22-21 correspond à pUC 19 contenant le *homing site* (43 pb) de l'intéine ou le site dans lequel le gène d'intéine est inséré.

La souche XL1-Blue [Tn10 *proA⁺B⁺ lacI^{q} Δ(lacZ)M15*/*recA1 endA1 gyrA96 (NaI^{r}) thi hsdR17 (r_{K}⁻m_{K}⁺)* supE44 *relA1 lac*] a été employée pour l'amplification de l'ADN plasmidique.

### 2) Réactifs.

Les enzymes de restriction et de modifications rapportées dans le tableau I ci-dessous ont été utilisées selon les recommandations des fournisseurs.

**Tableau I**

| Enzyme | Concentration | Fournisseur |
|---|---|---|
| *Sca I* | 10 U/µl | Appligene Oncor |
| *Sal I* | 8 U/µl | New England Biolabs |
| *Nde* I | 20 U/µl | New England Biolabs |
| *Bam* HI | 20 U/µl | New England Biolabs |
| *Eco* RI | 20 U/µl | New England Biolabs |
| T7 RNA polymérase | 50 U/µl | New England Biolabs |
| T4 DNA ligase | 400 U/µl | New England Biolabs |

Les tampons utilisés sont décrits dans le tableau II ci-dessous.

**Tableau II**

| Tampons | Composition |
|---|---|
| T | Tris HCl 10 mM pH 8,0 |
| T7 RNA polymérase (10X) | Tris HCl 400 mM pH 7,9, 60 mM MgC12, 20 mM spermidine, 100 mM DTT |
| IT2 (10X) | Tris-OAc 500 mM pH 8,0, 750 mM Mg(OAc)₂, 100 mM NH₄OAc |
| TADH (2X) | glycine 100 mM, NaOH pH 10, 20 mM butanol 1, 0,9 M NaCl, 4 mM NADP |
| LIP | KH₂PO₄ 0,1 M , pH 6, 8 , dioxane 5%, Thesit 5% |
| BETA | NaP 50 mM pH 7,0, 100 µg/ml nitrocéphine, 0,25 mM DMSO |
| Ligation 10X | Tris HCl 500 mM pH 7,5, 100 mM MgCl₂, 100 mM DTT, 10 mM ATP, 250 µg/ml BSA |

### II - Préparation et test des plasmides.

### 1) Constructions.

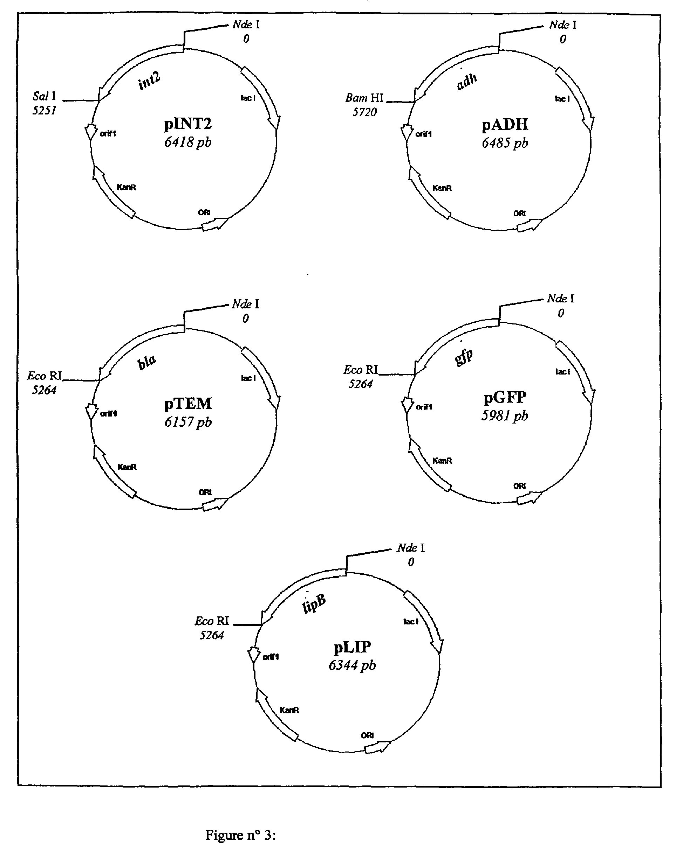

Le gène de l'intéine 2 de la Tfu ADN polymérase (itfu2) (numéro d'accession dans Genbank: Z69882) a été inséré entre les sites de restriction *Nde* I et *Sal* I du vecteur pET26b+ afin de créer le plasmide pET26-Tfu2 représenté à la figure 3. De la même façon, le gène de l'alcool deshydrogénase (*adh*) de *Thermococcus hydrothermalis* a été inséré entre les sites de restriction *Nde* I et *Bam* HI du vecteur pET26B+ pour créer le vecteur pADH, et les gènes de la β-lactamase TEM-1 (*bla*) *d'Escherichia coli* (9), et de la Green Fluorescent Protein (*gfp*) de *Aequorea victoria* (6) et de la lipase B de Candida antarctica (*lipB*) (numéro d'accession Y14015 dans Genbank) ont été insérés respectivement entre les sites de restriction *Nde* I et *Eco* RI du vecteur pET26b+, pour créer les plasmides pADH, pTEM, pGFP et pLIP représenté à la figure 3. Pour chacun de ces quatre gènes, le site de restriction *Nde* I est superposé avec le codon ATG de l'initiation de la traduction.

Chaque construction a été vérifiée par plusieurs analyses du profil de restriction. 200 µl de cellules XL1-Blue chimiocompétentes (1) ont été transformées avec 10 ng de chaque plasmide par un choc thermique (2), et les cellules ainsi transformées ont été étalées sur milieu LB solide contenant 60 µg/ml de kanamycine et 12,5 µg/ml de tétracycline. A partir d'un clone de chacune de ces transformations, une maxipréparation d'ADN plasmidique a été réalisée avec une colonne de type TIP100 (QIAGEN). Après précipitation à l'isopropanol, chaque ADN plasmidique a été repris dans 100 µl de tampon T. La concentration de ces ADN plasmidiques a été évaluée par un dosage spectrophotométrique à 260 nm ). La pureté de chaque ADN plasmidique a été vérifiée en déposant 0,2 µl de chacun des vecteurs sur gel d'agarose TBE 1%.

### 2) Tests in vivo de l'expression - tests de l'activité.

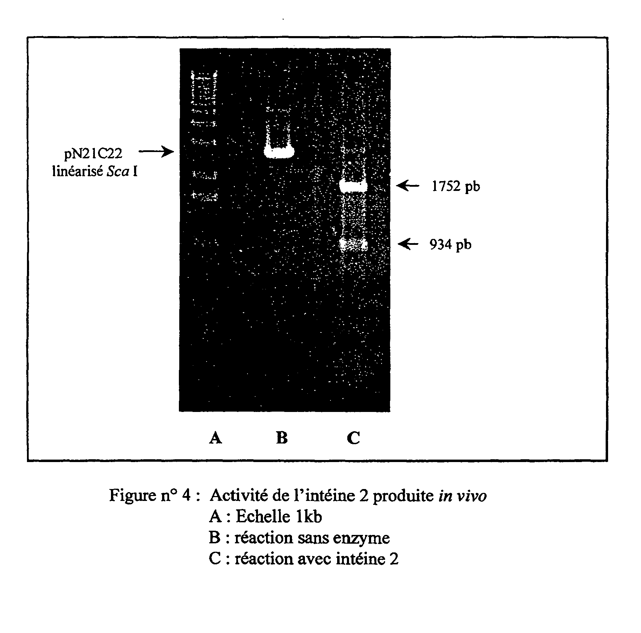

- pTEM : 200 µl de cellules BL21 DE3 (pLysS) chimiocompétentes ont été transformées avec 10 ng du plasmide pTEM par un choc thermique, et les cellules ainsi transformées ont été étalées sur milieu LB solide contenant 60 µg/ml de kanamycine, 20 µg/ml de chloramphénicol, 32 µg/ml d'IPTG et 100 µg/ml d'ampicilline. Après une nuit d'incubation à 37°C, de très nombreuses colonies ont pu être observées sur la boite de Pétri, révélant ainsi que le gène TEM-1 du plasmide pTEM est exprimé et fonctionnel, et qu'il confère une résistance à l'ampicilline.
- pGFP : 200 µl de cellules BL21 DE3 (pLysS) chimiocompétentes ont été transformées avec 10 ng du plasmide pGFP par un choc thermique, et les cellules ainsi transformées ont été étalées sur milieu LB solide contenant 60 µg/ml de kanamycine, 20 µg/ml de chloramphénicol et 32 µg/ml d'IPTG. Après une nuit d'incubation à 37°C, de très nombreuses colonies ont pu être observées sur la boite de Pétri. La totalité de ces colonies ont réagi à une excitation aux ultraviolets (à environ 400 nm) en émettant une lumière fluorescente verte, ce qui a permis de vérifier que le gène gfp du plasmide pGFP est exprimé et fonctionnel.
- pET26-Tfu2 : 200 µl de cellules BL21 DE3 (pLysS) chimiocompétentes ont été transformées avec 10 ng du plasmide pET26-Tfu2 par un choc thermique, et les cellules ainsi transformées ont été étalées sur milieu LB solide contenant 60 µg/ml de kanamycine et 20 µg/ml de chloramphénicol. Une culture réalisée à partir d'un clone de cette transformation a été induite à D0₆₀₀ₙₘ = 0,5 avec 0,5 mM d'IPTG pendant deux heures à 37°C. Après centrifugation, le culot bactérien a été repris dans du tampon phosphate de sodium 20 mM pH 7,5, et la lyse cellulaire a été obtenue grâce à plusieurs cycles dé congélation/décongélation. L'intéine 2 a ensuite été purifiée sur une colonne QFast-Flow avec un gradient de NaCl. L'activité de cette enzyme a été testée selon le protocole suivant:1 µl de la fraction d'élution la plus concentrée en enzyme pure a été diluée cent fois. Un µl de cette dilution a été incubé 15 minutes à 70°C avec 3 µl de tampon IT2 et 220 ng du plasmide pHS2-22-21, linéarisé avec l'enzyme de restriction *Sca* I, dans un volume final de 30 µl. 15 µl de ce mélange de digestion ont été déposés sur un gel d'agarose TBE 1%. Après migration et coloration au bromure d'éthidium, le gel a été exposé aux ultra violets. Comme montré à la figure 4, l'analyse de ce gel révèle la présence de deux bandes de respectivement 934 pb et 1752 pb, correspondant à la coupure du vecteur pHS2-22-21 (linéarisé *Sca* I) par l'intéine 2. Le gène *itfu2* du plasmide pET26-Tfu2 est donc exprimé et son produit, l'intéine 2, est actif.
- pADH : 200 µl de cellules BL21 DE3 (pLysS) chimiocompétentes ont été transformées avec 10 ng du plasmide pADH par un choc thermique, et les cellules ainsi transformées ont été étalées sur milieu LB solide contenant 60 (µg/ml de kanamycine et 20 µg/ml de chloramphénicol. Une culture réalisée à partir d'un clone de cette transformation a été induite à D0₆₀₀ₙₘ=0,6 avec 1 mM d'IPTG pendant trois heures à 37°C. Après centrifugation, le culot bactérien a été repris dans du tampon phosphate de sodium 50 mM - MgCl₂ 10 mM pH 8,0, et la lyse cellulaire a été obtenue en incubant 30 minutes sur la glace en présence de 1 mg/ml de lysozyme, 10 µg/ml de RNAse A et 100 µg/ml de DNAse I. Le surnageant de centrifugation de cette étape d'extraction a été incubé 30 minutes à 50°C, et centrifugé à nouveau. Le surnageant de cette dernière étape a servi d'extrait enzymatique pour les dosages d'activité. Un témoin négatif a été constitué en parallèle en réalisant une extraction similaire sur une culture de cellules BL21 DE3 (pLysS) ne portant aucun plasmide. L'activité alcool déshydrogénase a été testée en suivant spectrophotométriquement la cinétique de réduction du NADP en NADPH à 340 nm. Pour cela, 10 µl de l'extrait enzymatique, ou du témoin, ont été incubés à 50°C pendant cinq minutes avec 500 µl de tampon TADH et 490 µl d'eau. Dans ces conditions, une activité de 15,6 UDO/min/ml d'extrait enzymatique a été détectée, contre 0 UDO/min/ ml pour le témoin. Le gène *adh* du plasmide pADH est donc exprimé et son produit, l'alcool deshydrogénase, est actif.

### III - Essais d'Expression Protéique In Vitro (EPIV) avec un extrait de traduction préparé à partir de souches mésophiles (37°C).

4 µg de chaque vecteur ont été précipités en présence d'un dixième de volume d'acétate de sodium 3M pH 6,0, et de deux volumes d'éthanol absolu glacial. Les culots de précipitation ont été rincés à l'éthanol à 70% pour éliminer toute trace de sels. Chaque ADN précipité a été resuspendu dans 4 µl de tampon T.

Cet ADN est incubé deux heures à 37°C dans un mélange d'expression protéique *in vitro* contenant 0,1 mM de chacun des 20 acides aminés, 20 µl d'extrait "S30 Premix" et 15 µl de "T7 S30 extract" (PROMEGA) dans un volume final de 50 µl. Les extraits "S30 Premix" et "T7 S30 extract" contiennent tous les éléments nécessaires à une réaction de transcription *in vitro* couplée à une réaction de traduction, notamment : T7 ARN polymérase, CTP, UTP, GTP, ATP, tRNAs, EDTA, acide folique et sels appropriés. L'extrait de traduction a été réalisé selon la procédure décrite par Zubay (10) à partir d'une souche *d'Escherichia coli* B déficiente en endoprotéinase OmpT et en protéase lon, ce qui assure une meilleure stabilité des protéines exprimées *in vitro.*

Un témoin négatif a été constitué en incubant de l'eau stérile ultra pure à la place de l'ADN dans le mélange de transcription-traduction. Le témoin positif a été conçu en incubant 2 µg du plasmide PINPOINT^{™}.

Les réactions EPIV ont été conservées sur la glace jusqu'à ce que l'activité enzymatique de chaque échantillon ait pu être évaluée.

### IV - DOSAGE DES ACTIVITES.

### 1) Enzymes de mésophiles.

### a) Activité GFP.

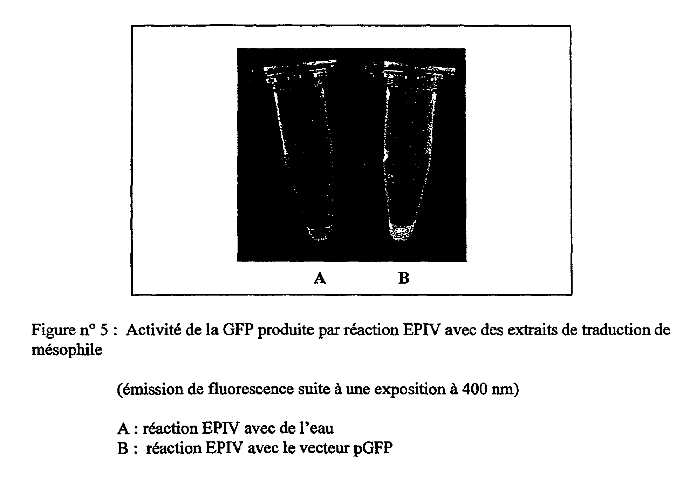

La figure 5 montre le tube contenant le produit de la réaction EPIV avec le vecteur pGFP qui a été exposé aux ultra violets à environ 400 nm, à côté du tube contenant la réaction témoin. Seul le tube contenant la réaction EPIV du plasmide pGFP a émis une lumière fluorescente verte. La réaction EPIV a donc permis la production d'une protéine GFP ayant une activité de fluorescence.

### b) Activité β-lactamase.

L'activité β-lactamase a été évaluée en suivant spectrophotométriquement la cinétique de dégradation de la nitrocéphine, une céphalosporine chromogénique, à 486 nm (5). Pour cela, 5, 10 ou 20 µl de la réaction EPIV avec le vecteur pTEM sont incubés 2 minutes à 37°C dans du tampon BETA qsp 1 ml. L'activité moyenne de la réaction EPIV avec le vecteur pTEM a pu être estimée à 8,9 UDO/min/ml d'extrait, contre 0,6 UDO/min/ ml d'extrait avec la réaction EPIV témoin. La réaction d'expression protéique *in vitro* a donc permis la synthèse d'une β-lactamase active, capable de dégrader la nitrocéphine *in vitro.*

Le vecteur témoin PINPOINT^{™} porte le gène *bla* sous contrôle de son promoteur endogène. Pourtant, l'activité β-lactamase de la réaction EPIV avec ce vecteur a été testée et évaluée à 6 UDO/min/ml d'extrait. Il convient de remarquer que l'ajout de rifampicine à 1 ng/µl, inhibiteur de l'ARN polymérase de *E. coli*, ne modifie pas significativement l'expression *in vitro* de la β-lactamase avec le vecteur PINPOINT^{™}. Le gène *bla* du vecteur PINPOINT^{™}, situé à 2123 pb en aval du promoteur T7 est donc transcrit et traduit efficacement. Ceci implique qu'un gène situé à 2000 pb en aval d'un promoteur T7 est efficacement transcrit et traduit lors d'une réaction EPIV.

### 2) Enzymes de thermophiles.

### a) Activité Intéine 2.

La réaction EPIV avec le vecteur pET26-Tfu2 a été testée afin de savoir si une intéine 2 active pouvait être produite. Pour cela, 5 µl de cette réaction EPIV ont été incubés 20 minutes à 70°C avec 220 ng de vecteur pHS2-22-21, linéarisé par *Sca* I, et 3 µl de tampon IT2 dans un volume final de 30 µl. Un témoin négatif a été constitué en remplaçant les 5 µl de la réaction EPIV par de l'eau. Le témoin positif contenait 1 µl de la fraction purifiée d'intéine 2 produite *in vitro* diluée au 1/100^{ème}. Enfin, un témoin de spécificité a été réalisé en incubant 5 µl de la réaction EPIV n'ayant pas reçu d'ADN.

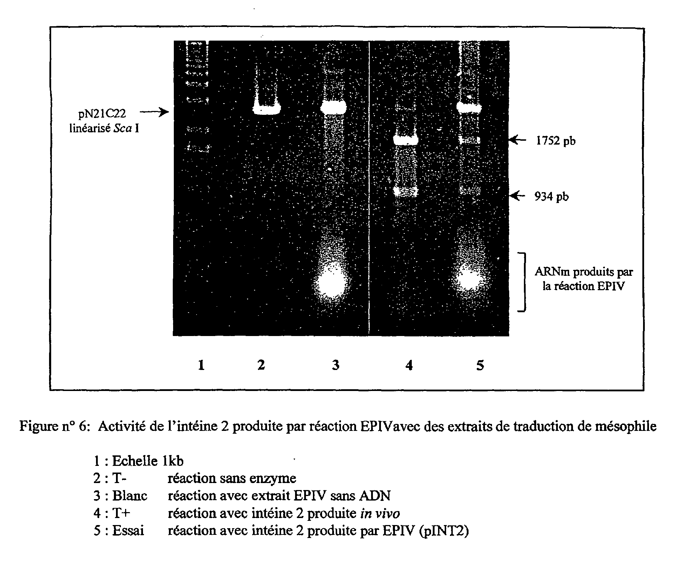

Après incubation, les quatre essais ont subi une extraction au phénol-chloroforme et une précipitation à l'éthanol absolu, suivie d'un rinçage à l'éthanol à 70 % afin d'éliminer les sels. Chaque culot de précipitation a été repris dans 10 µl de tampon T, et 8 µl ont été déposés sur un gel d'agarose TBE 1%. Après migration et coloration au bromure d'éthidium le gel a été exposé aux ultra violets pour analyser les profils de restriction. L'apparition de bandes à 934 et 1752 pb sur la piste 5 de la figure 6, identiques aux bandes de la piste 4 (témoin positif) révèle que l'intéine 2 est bien produite par la réaction EPIV, et que cette enzyme est active. De plus, la réaction EPIV est spécifique puisque aucune bande de digestion ne peut être observée sur le témoin de la piste 3.

### b) Activité alcool déshydrogénase.

L'activité alcool déshydrogénase a été testée en suivant spectrophotométriquement la cinétique de réduction du NADP en NADPH à 340 nm. Pour cela, 5, 10 ou 15 µl de la réaction EPIV avec le vecteur pADH, ou sans ADN, ont été incubés à 50°C pendant cinq minutes avec 500 µl de tampon TADH dans un volume final de 1 ml. Dans ces conditions, l'activité moyenne de la réaction EPIV avec le vecteur pADH a été estimée à 2,3 UDO/min/ml d'extrait, contre 0,32 UDO/min/ml d'extrait pour le témoin (réaction EPIV avec de l'eau). L'ADH de *Thermococcus hydrothermalis* (numéro d'accession Y14015 dans Genbank) a donc été produite sous forme active lors de la réaction EPIV.

### 3) Enzyme de psychrophile.

L'activité lipase a été testée en suivant spectrophotométriquement la cinétique de dégradation d'un lipide chromogénique (1,2-0-dilauryl-rac-glycero-3-glutaric acid-resorufin ester) à 572 nm. Pour cela, 5 µl de la réaction EPIV avec le vecteur pLIP, ou sans ADN ont été incubés à 70°C pendant quinze minutes dans le tampon de réaction en présence de 100 µg de substrat. Dans ces conditions, l'activité de la réaction EPIV avec le vecteur pLIP a été estimée à 0.50 UDO/min/ml d'extrait contre 0.04 UDO/min/ml d'extrait pour le témoin. La lipase B de *Candida* antarctica (organisme eucaryote) a donc été produite sous forme active lors de la réaction EPIV.

### V - Utilisation d'un extrait de traduction contenant une activité mésophile pour détecter une propriété thermophile.

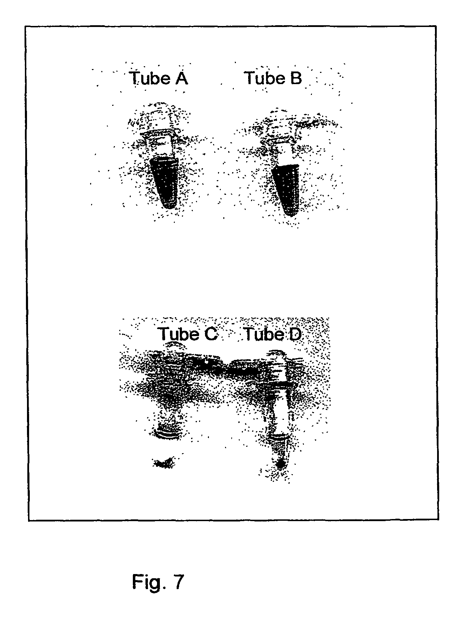

Le gène de la beta-galactosidase de *Thermotoga neapolitana* a été inséré dans un vecteur contenant le promoteur de transcription de la T7 RNA polymérase. Le vecteur ainsi obtenu a été utilisé pour réaliser une réaction EPIV avec un extrait de traduction d'une souche de *E.coli* possédant une activité béta-galactosidase. En parallèle, une réaction EPIV sans ADN a été effectuée. En incubant à 37°C une fraction de chacune des réactions EPIV en présence de Xgal à 37°C dans un tampon phosphate de sodium (50 mM, pH 7), les deux tubes ont viré au bleu en quelques minutes (cf figure 7 tubes A et B). En incubant une fraction de chacune des réactions EPIV en présence de Xgal dans les mêmes conditions mais à 70°C, seul le tube correspondant à la réaction EPIV avec le plasmide codant pour la béta-galactosidase thermophile a viré au bleu (cf figure 7 tubes C et D : ces deux tubes ont été centrifugés pour culotter les protéines de l'extrait de traduction mésophile qui ont précipité suite à leur dénaturation thermique durant le test d'activité à 70°C). L'activité béta-galactosidase mésophile n'est plus détectable à cette température (dénaturation thermique de la béta-galactosidse mésophile). Il est donc possible d'utiliser un extrait de traduction contenant une propriété similaire à la propriété recherchée si cette propriété n'est pas détectable dans les conditions de détection de la propriété recherchée.

### VI - Extrait de traduction préparés à partir d'organismes extrémophiles.

Des extraits de traduction d'autres organismes et en particulier d'organismes extrémophiles peuvent être préparés à partir de cellules selon l'une des procédures décrites par Zubay (1973) (10) ou par Pratt (1984) (7). Les vitesses de centrifugation, les conditions de cassage des cellules et les différents tampons de préparation ou de réaction seront ajustés à chaque type d'extrait de traduction par des essais systématiques. En réalisant ainsi une gamme d'extraits de traduction il devient possible de traduire un gène quelle que soit sont origine génétique : le procédé de l'invention permet donc d'avoir une adéquation entre le système de traduction et la ponctuation d'expression du gène codant pour la propriété recherchée.

### RÉFÉRENCES BIBLIOGRAPHIQUES

1) Hanahan D., (1985), Techniques for transformation of Escherichia coli, dans DNA cloning : a practical approach, Glover D.M. (Ed), IRL Press, Oxford vol 1, 109-135.
2) Maniatis T., Fristch E.F. et Sambrook J., (1982), Molecular cloning. A laboratory Manual, Cold Spring Harbor Laboratory, Cold Spring Harbor, NY.
3) Masson J.M., Meuris P., Grunstein M., Abelson J. et Miller H.J., (1987), Expression of a set of synthetic suppressor tRNAPhe genes in Saccharomyces cerevisiae, Proc. Natl. Acad. Sci. USA, 84, 6815-6819.
4) Normanly J., Masson J.-M., Kleina L.G., Abelson J. et Miller J.H., 1986, Construction of two Escherichia coli amber suppressor genes : tRNAPheCUA and tRNACysCUA, Proc. Natl. Acad. Sci. USA, 83, 6548-6552.
5) O'Callaghan C.H., Morris A., Kirby S.M. et Shingler A.H., 1972, Novel method for detection of beta-lactamases by using a chromogenic cephalosporin substrate, Antimicrob. Agents Chemother., 1, 283-288.
6) Prasher D.C., Eckenrode V.K., Ward W.W., Prendergast F.G et Cormier M.J., 1992, Primary structure of the Aequorea victoria green-fluorescent protein, Gene, 111, 229-233.
7) Pratt J.M., 1984, Coupled transcription-translation in prokaryotic cell-free systems, Transcription and Translation : A practical approach, Hames B.D. and Higgins S.J. Eds, JRL Press.
8) Studier F.W. et Moffatt B.A., 1986, Use of bacteriophage T7 RNA polymerase to direct selective high-level expression of cloned genes, J. Mol. Biol, 189, 113-130.
9) Sutcliffe J.G., 1978, Nucleotide sequence of the ampicillin résistance gene of Escherichia coli plasmid pBR322, Proc. Natl. Acad. Sci. USA, 75, 3737-3741.
10) Zubay G., 1973, In vitro synthesis of protein in microbial systems, Ann. Rev. Genet., 7, 267-287.

## Revendications

1. Procédé de criblage d'un échantillon biologique contenant des acides nucléiques, **caractérisé en ce qu'**il comprend les étapes suivantes :
a) la détermination de la fonction à cribler ,
b) la préparation de fragments d'acide nucléique à partir dudit échantillon,
c) l'association de chacun desdits fragments avec une molécule vectrice,
d) l'isolement de chaque fragment associé avec une molécule vectrice ou d'une partie de chaque construction constituée par un fragment associé avec une molécule vectrice, de l'étape (c)
e) le traitement dans un système acellulaire de chaque fragment associé avec une molécule vectrice ou d'une partie de chaque construction constituée par un fragment associé avec une molécule vectrice de l'étape (d) pour obtenir des transcrits,
f) le test de la fonction déterminée à l'étape (a) des transcrits obtenus à l'étape (e) ou des protéines pour lesquels ils codent après traduction desdits transcrits.

2. Procédé selon la revendication 1, **caractérisé en ce que** l'étape (f) comprend le traitement des transcrits obtenus à l'étape (e) *in vitro* avec un extrait cellulaire permettant leur traduction en protéine, puis le test d'une fonction desdites protéines par tout moyen approprié.

3. Procédé selon l'une quelconque des revendications précédentes, **caractérisé en ce que** l'échantillon correspond à un prélèvement brut.

4. Procédé selon l'une quelconque des revendications précédentes, **caractérisé en ce que** l'échantillon biologique comporte un ou plusieurs organismes.

5. Procédé selon la revendication 4, **caractérisé en ce que** les microorganismes de l'échantillon ne sont pas isolés.

6. Procédé selon l'une quelconque des revendications précédentes, **caractérisé en ce que** les acides nucléiques de l'échantillons sont inconnus.

7. Procédé selon l'une quelconque des revendications 1 à 6, **caractérisé en ce qu'**une banque d'ADN génomique est préparée à l'étape (b).

8. Procédé selon l'une quelconque des revendications 1 à 6, **caractérisé en ce qu'**une banque d'ADNc est préparée à l'étape (b).

9. Procédé selon l'une quelconque des revendications précédentes, **caractérisé en ce que** la molécule vectrice est composée d'une ou plusieurs séquences polynucléotidiques comportant au moins un promoteur de transcription pour l'étape (e) et éventuellement une substance facilitant l'isolement du fragment à l'étape (d).

10. Procédé selon l'une quelconque des revendications précédentes, **caractérisé en ce que** la molécule vectrice est composée de deux séquences polynucléotidiques comportant chacune au moins un promoteur de transcription, chacune de ces séquences étant associée à une extrémité d'un des fragments obtenus à l'étape (b).

11. Procédé selon l'une des revendications 9 ou 10, **caractérisé en ce que** le ou les promoteurs de transcription portés par la molécule vectrice sont de type fort.

12. Procédé selon l'une quelconque des revendications précédentes, **caractérisés en ce que** l'étape (e) de transcription est réalisée simultanément ou non avec la traduction de l'étape (f).

13. Procédé selon l'une quelconque des revendications 2 à 12, **caractérisé en ce que** l'extrait de traduction soit ne présente pas la fonction testée à l'étape (f), soit la présente mais alors n'est pas détectable dans les conditions du test réalisé à ladite étape (f).

14. Procédé selon l'une quelconque des revendications 2 à 13, **caractérisé en ce que** l'extrait de traduction est un mélange de plusieurs extraits possédants des caractéristiques différentes.

15. Procédé selon l'une quelconque des revendications 2 à 14, **caractérisé en ce que** l'extrait de traduction est complémenté par un ou plusieurs tRNAs présent en faible quantité ou absent de l'extrait utilisé, et/ou une ou plusieurs substances favorisant un repliement ou la maturation plus efficace des protéines exprimées.

16. Procédé selon l'une quelconque des revendications 2 à 15, **caractérisé en ce que** l'extrait de traduction est un extrait universel de traduction quelque soit l'origine de l'échantillon.

17. Procédé selon l'une quelconque des revendications précédentes, **caractérisé en ce que** la traduction du transcrit à l'étape (f) est réalisée avec un extrait de traduction de la même origine ou d'une origine proche de celle de l'échantillon biologique.

18. Procédé selon l'une quelconque des revendications précédentes, **caractérisé en ce que** l'étape (f) consiste à tester la fonction des transcrits comme ribozyme ou tRNA.

19. Procédé selon l'une quelconque des revendications 1 à 17, **caractérisé en ce que** l'étape (f) consiste à tester la fonction des protéines traduites desdits transcrits pour une activité enzymatique ou une affinité.

20. Procédé selon l'une quelconque des revendications précédentes, **caractérisé en ce que** l'étape (f) consiste à tester en parallèle ou de manière simultanée différentes propriétés d'une même fonction ou plusieurs fonctions.

21. Procédé selon l'une quelconque des revendications précédentes, **caractérisé en ce que** la molécule vectrice associée à chaque fragment de l'étape (c) est un vecteur plasmidique ne permettant préférentiellement pas l'expression dudit fragment *in vivo*.

22. Procédé selon l'une quelconque des revendications précédentes, **caractérisé en ce que** les promoteurs et éventuels terminateurs ne fonctionnent pas dans le microorganisme utilisé pour la séparation des vecteurs recombinants à l'étape (d).

23. Procédé selon l'une quelconque des revendications précédentes, **caractérisé en ce que** les fragments préparés à l'étape (b) ont une taille de 1 à plusieurs dizaine de kb, préférentiellement de 1 à 40 kb et avantageusement de 1 à 10 kb lorsque l'échantillon biologique provient d'un organisme procaryote.

24. Procédé selon la revendication 23, **caractérisé en ce que** les fragments préparés à l'étape (b) peuvent porter un opéron partiel ou entier.

25. Procédé selon l'une quelconque des revendications 1 à 22, **caractérisé en ce que** les fragments préparés à l'étape (b) ont avantageusement une taille de l'ordre de plusieurs dizaine à plusieurs centaines de kilobases dans le cas d'un organisme eucaryote.

26. Procédé selon la revendication 25, **caractérisé en ce que** l'échantillon biologique est une banque d'ADN eucaryote et **en ce que** la réaction de trancription de l'étape (e) est complétée par une réaction de splicing et de maturation in vitro des ARNm en utilisant un extrait nucléaire.

27. Procédé selon l'une quelconque des revendications précédentes, **caractérisé en ce que** la molécule vectrice associée aux fragments d'acide nucléique à l'étape (c) est un vecteur plasmidique, et **en ce que** à l'étape (c) chaque fragment est inséré dans un vecteur au niveau d'un site de clonage ou d'une cassette de restriction, ledit vecteur plasmidique comprenant :
- un promoteur d'ARN polymérase d'un côté du site de clonage et éventuellement un terminateur d'ARN polymérase de l'autre côté, ou
- un site de clonage encadré par deux promoteurs d'ARN polymérase identiques ou différents et éventuellement flanqués de part et d'autre du ou des terminateurs d'ARN polymérase correspondants.

28. Procédé selon l'une quelconque des revendications précédentes, **caractérisé en ce que** l'échantillon biologique provient d'un ou plusieurs organismes procaryotes ou cellules d'eucaryotes, ou encore de virus, identiques ou différentes.

29. Procédé selon l'une quelconque des revendications 1 à 22, **caractérisé en ce que** l'échantillon biologique est constitué d'une séquence ou d'une banque d'acides nucléiques synthétiques.

30. Procédé selon l'une des revendications précédentes, **caractérisé en ce que** l'isolement du vecteur recombinant à l'étape (d) est réalisé par transformation de cellules hôtes par l'ensemble des vecteurs recombinants obtenus à l'étape (c) de façon à créer une banque de clones, puis **en ce qu'**on réalise une extraction du vecteur ou dune partie du vecteur recombinant contenu par chaque clone de la banque par tout moyen approprié.

31. Procédé selon l'une quelconque des revendications précédentes, **caractérisé en ce qu'**il est réalisé entièrement sur un support solide de type puce ou membrane ou plaque de nanotitration.

32. Utilisation du procédé selon l'une quelconque des revendications précédentes, pour l'identification d'un fragment d'acide nucléique codant pour le transcrit ou la protéine possédant la fonction déterminée.

33. Utilisation selon la revendication 32, **caractérisée en ce qu'**elle comprend une étape supplémentaire de séquençage d'un fragment d'acide nucléique codant pour le transcrit ou la protéine possédant la fonction déterminée.

## Claims

1. Method for screening a biological sample containing nucleic acids, **characterized in that** it comprises the following steps:
a) determining the function to be screened,
b) preparing nucleic acid fragments from said sample,
c) associating each of said fragments with a vector molecule,
d) isolating each fragment associated with a vector molecule or a part of each construct consisting of a fragment associated with a vector molecule, of step (c),
e) treating, in an acellular system, each fragment associated with a vector molecule or a part of each construct consisting of a fragment associated with a vector molecule, of step (d), so as to obtain transcripts,
f) testing the function, determined in step (a), of the transcripts obtained in step (e) or of the proteins that they encode after translation of said transcripts.

2. Method according to Claim 1, **characterized in that** step (f) comprises treating the transcripts obtained in step (e), *in vitro,* with a cell extract which enables them to be translated into a protein, and then testing a function of said proteins by any appropriate means.

3. Method according to any one of the preceding claims, **characterized in that** the sample corresponds to a crude sample.

4. Method according to any one of the preceding claims, **characterized in that** the biological sample comprises one or more organisms.

5. Method according to Claim 4, **characterized in that** the microorganisms of the sample are not isolated.

6. Method according to any one of the preceding claims, **characterized in that** the nucleic acids of the sample are unknown.

7. Method according to any one of Claims 1 to 6, **characterized in that** a genomic DNA library is prepared in step (b).

8. Method according to any one of Claims 1 to 6, **characterized in that** a cDNA library is prepared in step (b).

9. Method according to any one of the preceding claims, **characterized in that** the vector molecule is composed of one or more polynucleotide sequences comprising at least one transcription promoter for step (e) and, optionally, a substance which facilitates the isolation of the fragment in step (d).

10. Method according to any one of the preceding claims, **characterized in that** the vector molecule is composed of two polynucleotide sequences each comprising at least one transcription promoter, each of these sequences being associated with one end of one of the fragments obtained in step (b).

11. Method according to either of Claims 9 and 10, **characterized in that** the transcription promoter(s) carried by the vector molecule is (are) of the strong type.

12. Method according to any one of the preceding claims, **characterized in that** the transcription step (e) is carried out simultaneously with or not simultaneously with the translation of step (f).

13. Method according to any one of Claims 2 to 12, **characterized in that** the translation extract does not comprise the function tested in step (f), or the function is present but cannot be detected under the conditions of the test carried out in said step (f).

14. Method according to any one of Claims 2 to 13, **characterized in that** the translation extract is a mixture of several extracts having different characteristics.

15. Method according to any one of Claims 2 to 14, **characterized in that** the translation extract is supplemented with one or more tRNAs present in a small amount or absent from the extract used, and/or one or more substances which promote folding or more effective maturation of the expressed proteins.

16. Method according to any one of Claims 2 to 15, **characterized in that** the translation extract is a universal translation extract regardless of the origin of the sample.

17. Method according to any one of the preceding claims, **characterized in that** the translation of the transcript in step (f) is carried out with a translation extract of the same origin as or of an origin close to that of the biological sample.

18. Method according to any one of the preceding claims, **characterized in that** step (f) consists in testing the function of the transcripts such as ribozyme or tRNA.

19. Method according to any one of Claims 1 to 17, **characterized in that** step (f) consists in testing the function of the proteins translated from said transcripts for enzymatic activity or affinity.

20. Method according to any one of the preceding claims, **characterized in that** step (f) consists in testing, in parallel or in a simultaneous manner, various properties of the same function or several functions.

21. Method according to any one of the preceding claims, **characterized in that** the vector molecule associated with each fragment of step (c) is a plasmid vector which preferably does not allow said fragment to be expressed *in vivo.*

22. Method according to any one of the preceding claims, **characterized in that** the promoters and optional terminators are not functional in the microorganism used for separating the recombinant vectors in step (d).

23. Method according to any one of the preceding claims, **characterized in that** the fragments prepared in step (b) have a size of from 1 to several tens of kb, preferably from 1 to 40 kb, and advantageously from 1 to 10 kb, when the biological sample originates from a prokaryotic organism.

24. Method according to Claim 23, **characterized in that** the fragments prepared in step (b) can carry a partial or entire operon.

25. Method according to any one of Claims 1 to 22, **characterized in that** the fragments prepared in step (b) advantageously have a size of the order of several tens to several hundreds of kilobases in the case of a eukaryotic organism.

26. Method according to Claim 25, **characterized in that** the biological sample is a eukaryotic DNA library and **in that** the transcription reaction in step (e) is completed by a reaction for splicing and maturation, *in vitro,* of the mRNAs using a nuclear extract.

27. Method according to any one of the preceding claims, **characterized in that** the vector molecule associated with the nucleic acid fragments in step (c) is a plasmid vector, and **in that**, in step (c), each fragment is inserted into a vector at the level of a cloning site or of a restriction cassette, said plasmid vector comprising:
- an RNA polymerase promoter on one side of the cloning site and, optionally, an RNA polymerase terminator on the other side, or
- a cloning site flanked by two identical or different RNA polymerase promoters and, optionally, flanked on both sides by the corresponding RNA polymerase terminators.

28. Method according to any one of the preceding claims, **characterized in that** the biological sample originates from one or more prokaryotic organisms or eukaryotic cells, or viruses, which may be identical or different.

29. Method according to any one of Claims 1 to 22, **characterized in that** the biological sample consists of a sequence or a library of synthetic nucleic acids.

30. Method according to one of the preceding claims, **characterized in that** the isolation of the recombinant vector in step (d) is carried out by transforming host cells with all the recombinant vectors obtained in step (c) in such a manner as to create a clone library, and then **in that** the recombinant vector or a part of the recombinant vector contained in each clone of the library is extracted by any appropriate means.

31. Method according to any one of the preceding claims, **characterized in that** it is carried out entirely on a solid support of the chip, membrane or nanotitration plate type.

32. Use of the method according to any one of the preceding claims, for identifying a nucleic acid fragment encoding the transcript or the protein having the determined function.

33. Use according to Claim 32, **characterized in that** it comprises an additional step of sequencing a nucleic acid fragment encoding the transcript or the protein having the determined function.

## Patentansprüche

1. Verfahren zum Screening einer biologischen Probe, die Nukleinsäuren enthält, **dadurch gekennzeichnet, dass** es die folgenden Schritte umfasst:
a) Bestimmen der zu screenenden Funktion,
b) Herstellen von Nukleinsäurefragmenten aus der Probe,
c) Verbinden jedes der Fragmente mit einem Vektormolekül,
d) Isolieren jedes mit einem Vektormolekül verbundenen Fragments oder eines Teils jeder Konstruktion, die aus einem mit einem Vektormolekül verbundenen Fragment besteht, des Schrittes (c),
e) Behandeln jedes mit einem Vektormolekül verbundenen Fragments oder eines Teils jeder Konstruktion, die aus einem mit einem Vektormolekül verbundenen Fragment besteht, des Schrittes (d) in einem zellfreien System, um Transkripte zu erhalten,
f) Testen der im Schritt (a) bestimmten Funktion der im Schritt (e) erhaltenen Transkripte oder der Proteine, für die sie codieren, nach Translation der Transkripte.

2. Verfahren nach Anspruch 1, **dadurch gekennzeichnet, dass** der Schritt (f) die *In-vitro-*Behandlung der im Schritt (e) erhaltenen Transkripte mit einem Zellextrakt, der ihre Translation in Proteine gestattet, und anschließend das Testen einer Funktion der Proteine durch jedes geeignete Mittel umfasst.

3. Verfahren nach einem der vorhergehenden Ansprüche, **dadurch gekennzeichnet, dass** die Probe einer rohen Probennahme entspricht.

4. Verfahren nach einem der vorhergehenden Ansprüche, **dadurch gekennzeichnet, dass** die biologische Probe einen oder mehrere Organismen enthält.

5. Verfahren nach Anspruch 4, **dadurch gekennzeichnet, dass** die Mikroorganismen der Probe nicht isoliert werden.

6. Verfahren nach einem der vorhergehenden Ansprüche, **dadurch gekennzeichnet, dass** die Nukleinsäuren der Proben unbekannt sind.

7. Verfahren nach einem der Ansprüche 1 bis 6, **dadurch gekennzeichnet, dass** im Schritt (b) eine genomische DNA-Bank hergestellt wird.

8. Verfahren nach einem der Ansprüche 1 bis 6, **dadurch gekennzeichnet, dass** im Schritt (b) eine cDNA-Bank hergestellt wird.

9. Verfahren nach einem der vorhergehenden Ansprüche, **dadurch gekennzeichnet, dass** das Vektormolekül aus einer oder mehreren Polynukleotidsequenzen besteht, die mindestens einen Transkriptionspromotor für den Schritt (e) und gegebenenfalls eine Substanz enthalten, welche die Isolation des Fragments im Schritt (d) erleichtert.

10. Verfahren nach einem der vorhergehenden Ansprüche, **dadurch gekennzeichnet, dass** das Vektormolekül aus zwei Polynukleotidsequenzen besteht, die jeweils mindestens einen Transkriptionspromotor enthalten, wobei jede dieser Sequenzen an einem Ende mit einem der im Schritt (b) erhaltenen Fragmente verbunden ist.

11. Verfahren nach einem der Ansprüche 9 oder 10, **dadurch gekennzeichnet, dass** der oder die in dem Vektormolekül enthaltenen Transkriptionspromotoren starke Promotoren sind.

12. Verfahren nach einem der vorhergehenden Ansprüche, **dadurch gekennzeichnet, dass** der Transkriptionsschritt (e) gleichzeitig mit der Translation des Schrittes (f) durchgeführt wird oder nicht.

13. Verfahren nach einem der Ansprüche 2 bis 12, **dadurch gekennzeichnet, dass** der Translationsextrakt entweder die im Schritt (f) getestete Funktion nicht aufweist oder sie aufweist, wobei sie aber unter den Bedingungen des im Schritt (f) durchgeführten Tests nicht nachweisbar ist.

14. Verfahren nach einem der Ansprüche 2 bis 13, **dadurch gekennzeichnet, dass** der Translationsextrakt ein Gemisch mehrerer Extrakte mit unterschiedlichen Eigenschaften ist.

15. Verfahren nach einem der Ansprüche 2 bis 14, **dadurch gekennzeichnet, dass** der Translationsextrakt mit einer oder mehreren tRNAs, die in dem verwendeten Extrakt in kleiner Menge vorhanden sind oder fehlen, und/oder einer oder mehreren Substanzen angereichert wird, welche eine Faltung oder effizientere Reifung der exprimierten Proteine begünstigen.

16. Verfahren nach einem der Ansprüche 2 bis 15, **dadurch gekennzeichnet, dass** der Translationsextrakt ein universeller Translationsextrakt ungeachtet des Ursprungs der Probe ist.

17. Verfahren nach einem der vorhergehenden Ansprüche, **dadurch gekennzeichnet, dass** die Translation des Trans-kripts des Schrittes (f) mit einem Translationsextrakt aus der gleichen Quelle oder aus einer ähnlichen Quelle wie die biologische Probe durchgeführt wird.

18. Verfahren nach einem der vorhergehenden Ansprüche, **dadurch gekennzeichnet, dass** der Schritt (f) das Testen der Funktion der Transkripte als Ribozym oder tRNA umfasst.

19. Verfahren nach einem der Ansprüche 1 bis 17, **dadurch gekennzeichnet, dass** der Schritt (f) das Testen der Funktion der translatierten Proteine dieser Transkripte auf eine enzymatische Aktivität oder eine Affinität umfasst.

20. Verfahren nach einem der vorhergehenden Ansprüche, **dadurch gekennzeichnet, dass** der Schritt (f) das parallele oder gleichzeitige Testen verschiedener Eigenschaften einer gleichen Funktion oder mehrerer Funktionen umfasst.

21. Verfahren nach einem der vorhergehenden Ansprüche, **dadurch gekennzeichnet, dass** das mit jedem Fragment des Schrittes (c) verbundene Vektormolekül ein Plasmidvek-tor ist, der vorzugsweise die In-vivo-Expression des Fragments nicht gestattet.

22. Verfahren nach einem der vorhergehenden Ansprüche, **dadurch gekennzeichnet, dass** die Promotoren und die ebventuellen Terminatoren nicht in dem Mikroorganismus funktionell sind, der zur Trennung der rekombinanten Vektoren des Schrittes (d) verwendet wird.

23. Verfahren nach einem der vorhergehenden Ansprüche, **dadurch gekennzeichnet, dass** die im Schritt (b) hergestellten Fragmente eine Größe von 1 bis zu mehreren zehn kb, vorzugsweise von 1 bis 40 kb und vorteilhafterweise von 1 bis 10 kb aufweisen, wenn die biologische Probe aus einem prokaryotischen Organismus stammt.

24. Verfahren nach Anspruch 23, **dadurch gekennzeichnet, dass** die im Schritt (b) hergestellten Fragmente ein partielles oder gesamtes Operon tragen können.

25. Verfahren nach einem der Ansprüche 1 bis 22, **dadurch gekennzeichnet, dass** die im Schritt (b) hergestellten Fragmente im Fall eines eukaryotischen Organismus vorteilhafterweise eine Größe in der Größenordnung von mehreren zehn bis mehreren hundert Kilobasen aufweisen.

26. Verfahren nach Anspruch 25, **dadurch gekennzeichnet, dass** die biologische Probe eine eukaryotische DNA-Bank ist und dass die Transkriptionsreaktion im Schritt (e) durch eine *In-vitro-*Spleiß- und -Reifungsreaktion der mRNAs unter Verwendung von Kemextrakt vervollständigt wird.

27. Verfahren nach einem der vorhergehenden Ansprüche, **dadurch gekennzeichnet, dass** das mit den Nukleinsäurefragmenten des Schrittes (c) verbundene Vektormolekül ein Plasmidvektor ist und dass im Schritt (c) jedes Fragment in einen Vektor in eine Klonierungsstelle oder eine Restriktionskassette inseriert wird, wobei der Plasmidvektor Folgendes umfasst:
- einen RNA-Polymerase-Promotor auf einer Seite der Klonierungsstelle und gegebenenfalls einen RNA-Polymerase-Terminator auf der anderen Seite, oder
- eine Klonierungsstelle, die von zwei gleichen oder verschiedenen RNA-Polymerase-Promotoren eingerahmt wird, die gegebenenfalls auf beiden Seiten von dem oder den entsprechenden RNA-Polymerase-Terminatoren flankiert werden.

28. Verfahren nach einem der vorhergehenden Ansprüche, **dadurch gekennzeichnet, dass** die biologische Probe von eine(m/r) oder mehreren prokaryotischen Organismen oder eukaryotischen Zellen oder auch von gleichen oder verschiedenen Viren stammt.

29. Verfahren nach einem der Ansprüche 1 bis 22, **dadurch gekennzeichnet, dass** die biologische Probe aus einer synthetischen Sequenz oder einer Bank synthetischer Nukleinsäuren stammt.

30. Verfahren nach einem der vorhergehenden Ansprüche, **dadurch gekennzeichnet, dass** die Isolation des rekombinanten Vektors im Schritt (d) durch Transformation von Wirtszellen mit der Gesamtheit der im Schritt (c) erhaltenen rekombinanten Vektoren, so dass eine Klonbank hergestellt wird, und anschließende Extraktion des in jedem Klon der Bank enthaltenen rekombinanten Vektors oder eines Teils des rekombinanten Vektors durch jedes geeignete Mittel durchgeführt wird.

31. Verfahren nach einem der vorhergehenden Ansprüche, **dadurch gekennzeichnet, dass** es vollständig auf einem festen Träger des Typs eines Chips oder einer Membran oder einer Nanotiterplatte durchgeführt wird.

32. Verwendung des Verfahrens nach einem der vorhergehenden Ansprüche zur Identifikation eines Nukleinsäurefragments, das für das Transkript oder das Protein mit der bestimmten Funktion codiert.

33. Verwendung nach Anspruch 32, **dadurch gekennzeichnet, dass** sie einen zusätzlichen Schritt des Sequenzierens eines Nukleinsäurefragments, das für das Transkript oder das Protein mit der bestimmten Funktion codiert, umfasst.
